(19) Europäisches Patentamt
European Patent Office
Office européen des brevets

(11) **EP 2 198 770 A1**

(12) **EUROPEAN PATENT APPLICATION**

(43) Date of publication:
**23.06.2010 Bulletin 2010/25**

(51) Int Cl.:
**A61B 3/032** (2006.01)  **A61B 5/103** (2006.01)
**A61B 5/11** (2006.01)

(21) Application number: **08305974.1**

(22) Date of filing: **18.12.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicants:
• **Essilor International
(Compagnie Générale D'Optique)
94220 Charenton le Pont (FR)**
• **UNIVERSITE PARIS-SUD (PARIS 11)
91405 Orsay (FR)**

(72) Inventors:
• **Amorim, Michel-Ange
c/o UNIVERSITE PARIS SUD (Paris 11)
91405 Orsay (FR)**

• **Fourre, Benoit
c/o UNIVERSITE PARIS SUD (Paris 11)
91405 Orsay (FR)**
• **Isableu, Brice
c/o UNIVERSITE PARIS SUD (Paris 11)
91405 Orsay (FR)**
• **Giraudet, Guillaume
c/o ESSILOR INTERNATIONAL
94220 Charenton le Pont (FR)**

(74) Representative: **Cabinet Plasseraud
52, rue de la Victoire
75440 Paris Cedex 09 (FR)**

(54) **Method for measuring the perception of spatial orientation of a person**

(57) A method for measuring the perception of spatial orientation of a person comprising the steps of:
- modifying a first non visual frame of reference and providing a second non visual frame of reference and measuring the value of the parameter representative of the perception of spatial orientation;
- modifying a first visual frame of reference so as to provide a second visual frame of reference and measuring the value of the parameter representative of the perception of spatial orientation;
- combining the measured values of the parameters representative of the perception of spatial orientation of preceding steps so as to obtain a value representing the sensitivity of the person referred to a scale of sensitivity to perception of spatial orientation.

FIG.2.

# EP 2 198 770 A1

**Description**

The present invention relates to a method for measuring the perception of spatial orientation of a person. Spatial orientation is the ability to maintain the body orientation and/or posture in relation with environment (physical space) at rest and during motion.

Spatial orientation relies for example on the use of visual, vestibular (organs of equilibrium located in the inner ear), proprioceptive (receptors located in the skin, muscles, tendons, and joints), auditory, sensory information.

Spatial orientation thus relates to the capacity to assess the physical relationship between the body and the environment, and to deal with modifications in this relationship during movement.

In order to determine the perception of spatial orientation ability of a person, one can choose a frame of reference for a sensory information type and measure the sensitivity of the person when the frame of reference for said sensory information varies.

One can thus determine for example the sensitivity to visual, or vestibular, or proprioceptive, or auditory information.

Prior knowledge is another frame of reference that can be studied and which relates to the knowledge the person already has before they meet new information.

According to examples the proprioceptive sensitivity, which relates to the unconscious perception of movement and spatial orientation arising from stimuli within the body, is measured in tests that measure the subject's ability to detect an externally imposed passive movement, or the ability to reposition a joint or a part of the body to a predetermined position. Error in the detection of the posture of the body relative to real gravity or error in the perception of what the true vertical of the environment is, may be measured so as to quantify the proprioceptive sensitivity.

Even though the spatial orientation sensitivity of a person can be estimated or measured within the different frames of reference, there remains still a need for measuring the perception of spatial orientation of a person in complex situations.

Thus the goal of the present invention is to provide a method for measuring the perception of spatial orientation of a person in complex situations, corresponding for example to virtual reality sickness (also called cybersickness or barfogenesis), transport sickness, pilots behaviour, sport training situations, handicap treatment such as sensory replacement, substitution or rehabilitation.

This object is obtained according to the invention by a method for measuring the perception of spatial orientation of a person comprising the steps of:

a1) measuring the value of a parameter representative of the perception of spatial orientation when the person's body is arranged according to a first non visual frame of reference;

a2) modifying the first non visual frame of reference and thus providing a second non visual frame of reference and measuring the value of the parameter representative of the perception of spatial orientation;

b1) measuring the value of the parameter representative of the perception of spatial orientation when the person's eyes are provided with a first visual frame of reference;

b2) modifying the first visual frame of reference so as to provide a second visual frame of reference and measuring the value of the parameter representative of the perception of spatial orientation;

c) combining the measured values of the parameters representative of the perception of spatial orientation of steps a1), a2), b1), b2) so as to obtain a value representing the sensitivity of the person referred to a scale of sensitivity to perception of spatial orientation.

According to the present invention and thanks to measuring the value of a parameter representative of the perception of spatial orientation according both to a non visual frame of reference and to a visual frame of reference and then combining both measured values so as to obtain a value representing the sensitivity of the person referred to a scale of sensitivity, one can quantify the perception of spatial orientation of a person in complex situations.

According to an embodiment of the present invention, the non visual frame of reference is selected in the list consisting of a proprioceptive frame of reference, a vestibular frame of reference, a non visual prior knowledge frame of reference, an auditory frame of reference.

According to an embodiment of the present invention, the non visual frame of reference is a proprioceptive frame of reference and following embodiments may be implemented and may be combined according to all possible combinations:

2

- modifying a proprioceptive frame of reference comprises modifying an exocentered spatial mass repartition of the parts of the body of the person;

- the variation of the exocentered spatial mass repartition of the parts of the body of the person is provided by adding at least a weight to at least a body part of the person;

- the part of the body to which at least a weight is attached is chosen in the list consisting of the head, the shoulder (s), part(s) of the trunk such as the dorsal region, the lumbar region;

- the variation of the exocentered spatial mass repartition of the parts of the body of the person is provided by inclining the supporting surface of the person;

- the variation of the exocentered spatial mass repartition of the parts of the body of the person is a variation between two static positions.

- the variation of the exocentered spatial mass repartition of the parts of the body of the person is a dynamic variation;

- modifying a proprioceptive frame of reference comprises providing a vibratory stimulation to the muscles and/or the tendons of the person.

According to an embodiment of the present invention, the non visual frame of reference is a vestibular frame of reference and where modifying a vestibular frame of reference comprises providing an electrical and/or a caloric stimulation to the ear region of the person.

According to an embodiment of the present invention, the non visual frame of reference is a non visual prior frame of reference and where modifying a prior knowledge frame of reference comprises providing verbal instructions to the person.

According to an embodiment of the present invention, providing a first visual frame of reference and modifying the first visual frame of reference so as to provide a second visual frame of reference comprises providing a first visual scene and modifying the first visual scene dynamically.

According to another embodiment of the present invention, providing a first visual frame of reference consists in providing a first static visual scene and providing a second visual frame of reference consists in providing a second static visual scene.

According to an embodiment of the present invention, providing a first visual frame of reference and modifying the first visual frame of reference so as to provide a second visual frame of reference comprises providing a first visual scene and tilting and/or shifting the first visual scene.

According to an embodiment of the present invention, the visual frame of reference is provided by projecting a three dimensional virtual visual scene on a screen.

According to an embodiment of the present invention, the three dimensional virtual visual scene comprises lines that are recognizable by the person as horizontal or vertical lines.

According to said embodiment the three dimensional virtual visual scene is an architectural or a landscape scene.

According to an embodiment of the present invention, the parameter representative of the perception of spatial orientation is the difference between at least a perceived and an actual vertical line.

According to said embodiment, the measuring method of the parameter representative of the perception of spatial orientation is a Rod and Frame Test. The Rod and Frame Test (RTF) is one of the key measures of the cognitive style construct of field-dependence-independence. During the test, observers view a tilted square frame which takes up most of the visual field, and an adjustable rod which tilts on the same center as the frame. They are asked to adjust the rod to the gravitational vertical, and these adjustments vary greatly (see Oltman PK.A portable rod-and-frame apparatus, Percept. Mot. Skills 26:503-6, 1968 *[*Psychol. Lab., Dept. Psychiat., State Univ. New York Downstate Medical Center, Brooklyn, NY*]* and also Witkin HA, Goodenough DR & Oltman P K. Psychological differentiation: current status. J. Personal. Soc. Psychol. 37:1127-45, 1979*)*.

According to an example, the measured parameter is the deviation error of a rod between the perceived and the actual vertical or horizontal. The measured parameter can also be the deviation error of a rod between the perceived and actual axis of a part of the body, such as the head or the trunk. In order to obtain a mean value and a variance value for the measured parameter, one provides for example on a computer screen a serie of rod orientation shifted to the right and to the left from the actual vertical and the person has to use the mouse or keys of the keyboard to indicate the perceived spatial orientation. A deviation error is calculated for each rod orientation and the *"just noticeable difference"* is calculated so as to estimate the variance value and the *"point of subjective equality"* is calculated so as to estimate the deviation error mean value.

The measuring of the perception of spatial orientation may be implemented according to following embodiments that may be combined:

- the measuring steps of steps a1) and a2) are implemented without providing any visual scene to the person;

- the steps a1) and a2) are implemented with the same visual scene provided to the person, the steps b1) and b2) are implemented with the same non visual frame of reference provided to the person and the combination of measured values of step c) is implemented according to a multisensory integration model; according to an example the multisensory integration model is selected in the list consisting of a Bayesian model, a Maximum Likelihood Estimation (MLE) model, a "winner-take-all" model;

- the steps a2) and b2) are implemented simultaneously; a modification of both visual and non visual frames of reference is thus provided simultaneously;

- both preceding embodiments are implemented and the result of the combination of the measured values of step c) where step a1) and a2) are implemented with the same visual scene and b1) and b2) implemented with the same non visual frame of reference is further compared with the result of the combination of the measured values of step c) when steps a2) and b2) are implemented simultaneously so as to test, and possibly correct, the result of the multisensory integration model.

- a plurality of non visual frames of reference is chosen and the steps a1 and a2 are implemented for each chosen non visual frame of reference; a plurality of non visual frame of reference can then be provided to the person and their influence on the perception of spatial orientation can be measured for said person;

- steps a2, b2, c) are repeated with a plurality of modified non visual frame of reference for a same type of frame of reference; it is then possible to determine for example perception of spatial orientation threshold, when the amplitude of the modification of frame of reference is progressively increased;

Bayesian probability interprets the concept of probability as "*a measure of a state of knowledge*". The term "*Bayesian*" refers to Thomas Bayes (1702-1761) who proved a special case of what is now called Bayes' theorem. Laplace proved a more general version of the theorem and used it to approach problems in celestial mechanics, medical statistics and reliability. Bayesian probability interprets *"probability"* as "*the degree of belief (or strength of belief) an individual has in the truth of a proposition*" and is in that respect subjective.

Maximum Likelihood Estimation (MLE) is a popular statistical method used for fitting a mathematical model to some data. The modelling of real world data using estimation by maximum likelihood offers a way of tuning the free parameters of the model to provide a good fit. For a fixed set of data and underlying probability model, maximum likelihood picks the values of the model parameters that make the data "more likely" than any other values of the parameters would make them. Maximum likelihood estimation gives a unique and easy way to determine solution in the case of the normal distribution and many other problems, although in very complex problems this may not be the case. If a uniform prior distribution is assumed over the parameters, the maximum likelihood estimate coincides with the most probable values thereof.

According to an embodiment of the present invention, "*combining the measured values of the parameters representative of the perception of spatial orientation of steps a1), a2), a3), a4)*" comprises:

c1) calculating a variation index (VI NV) for the parameter representative of the perception of spatial orientation according to the non visual frame of reference modifications (resulting from steps a1) and a2)) and calculating a variation index (VI_V) for the parameter representative of the perception of spatial orientation according to visual frame of reference modification (resulting from steps b1) and b2));

c2) obtaining the value representing the sensitivity of the person by calculating a relative variation between the variation index (VI_V) according to visual frame of reference modification and the variation index (VI_NV) according to non visual frame of reference modification.

According to preceding embodiment, calculating a variation index (VI) according to a frame of reference modification in step c1), consists in calculating the ratio between the amplitude of the variation of the value of the parameter representative of the perception of spatial orientation and the amplitude of the modification of the frame of reference, between a2) and a1) to calculate VI_NV, and between b2) and b1) to calculate VI_V.

According to an embodiment, calculating the relative variation between the variation indexes according to respectively visual and non visual frame of reference modifications, consists in calculating a ratio between VI_V and VI_NV.

According to an embodiment of the present invention, the scale of sensitivity to perception of spatial orientation is a numeral scale where the higher is the value representing the sensitivity of the person, the higher is the visual dependence compared to the non visual dependence according to the non visual frame of reference of steps a1), a2).

According to an example, the calculation of the relative variation between the variation indexes according to respectively visual and non visual frame of reference modifications comprises a normalisation step so as the scale of sensitivity to perception of spatial orientations varies from 0 to 1, where a 0 value corresponds to no visual dependence and 1 corresponds to a total visual dependence.

It has to be enlightened that the present invention is not limited to the exemplified scale of sensitivity and that scales of sensitivity using a non numeral scale can be used (such as letters, for example A, B, C, D, E, linked to a sensitivity level). Furthermore, the direction of the variation is not limited from low, respectively high, sensitivity value corresponding to low, respectively high, visual dependence but can be for example opposite.

According to an example the value SV representing the sensitivity of the person referred to a scale of sensitivity to perception of spatial orientation is calculated according to following equation (1) where one non visual frame of reference influence is tested:

$$SV = \frac{VI\_V \; / \; VI\_NV}{1 \; + \; (VI\_V \; / \; VI\_NV)} \qquad (1)$$

Corresponding scale of sensitivity varies from 0 to 1, and when SV is low, respectively high, the person has a low, respectively high, visual dependence.

According to another example, where a plurality of non visual frame of reference is tested the values SV representing the sensitivity of the person is calculated according to following equation (2):

$$SV = \frac{VI\_V \; / \; (w1.VI\_NV1 \; + \; \ldots \; + \; wn.VI\_NVn)}{1 \; + \; (VI\_V \; / \; (w1.VI\_NV1 \; + \; \ldots \; + \; wn.VI\_NVn)} \qquad (2)$$

Where:

n is the number of non visual frames of reference that are tested;

VI_NV1 to VI_NVn are the variation indexes according to the different tested non visual frames of reference;

w1 to wn are weight values used to take into account the importance of each of the non visual frame of reference; according to an example, each weight values are equal to 1;

According to an example n=4 and VI_NV1, VI_NV2, VI_NV3, VI_NV4 corresponds to the variation index according to respectively a proprioceptive, a vestibular, a non visual prior knowledge, an auditory frame of reference.

The invention also relates to a virtual reality helmet comprising a projection device wherein bars are fixed on said helmet and are suitable to receive weights.

The invention also relates to a computer program product comprising one or more stored sequences of instructions that are accessible to a processor and which, when executed by the processor, causes the processor to carry out the steps of the preceding methods.

The invention also relates to a computer-readable medium carrying one or more sequences of instructions of the computer program product.

Unless specifically stated otherwise, as apparent from the following discussions, it is appreciated that throughout the specification discussions utilizing terms such as "computing", "calculating" "generating", or the like, refer to the action and/or processes of a computer or computing system, or similar electronic computing device, that manipulate and/or transform data represented as physical, such as electronic, quantities within the computing system's registers and/or memories into other data similarly represented as physical quantities within the computing system's memories, registers or other such information storage, transmission or display devices.

Embodiments of the present invention may include apparatuses for performing the operations herein. This apparatus may be specially constructed for the desired purposes, or it may comprise a general purpose computer or Digital Signal Processor ("DSP") selectively activated or reconfigured by a computer program stored in the computer. Such a computer program may be stored in a computer readable storage medium, such as, but is not limited to, any type of disk including floppy disks, optical disks, CD-ROMs, magnetic-optical disks, read-only memories (ROMs), random access memories (RAMs) electrically programmable read-only memories (EPROMs), electrically erasable and programmable read only memories (EEPROMs), magnetic or optical cards, or any other type of media suitable for storing electronic instructions, and capable of being coupled to a computer system bus.

The processes and displays presented herein are not inherently related to any particular computer or other apparatus. Various general purpose systems may be used with programs in accordance with the teachings herein, or it may prove convenient to construct a more specialized apparatus to perform the desired method. The desired structure for a variety of these systems will appear from the description below. In addition, embodiments of the present invention are not described with reference to any particular programming language. It will be appreciated that a variety of programming languages may be used to implement the teachings of the inventions as described herein.

The features of the present invention, as well as the invention itself, both as to its structure and its operation, will be best understood from the accompanying non limiting drawings and examples, taken in conjunction with the accompanying description, in which :

- figure 1 shows an example of means to modify the proprioceptive frame of reference of a person;

- figure 2 shows an example of combining proprioceptive and visual frames of reference modifications;

- figures 3 and 4 show other examples of means to modify the proprioceptive frame of reference of a person;

- figures 5a to d show different configurations of a virtual reality helmet according to the present invention.

Skilled artisans appreciate that elements in the figures are illustrated for simplicity and clarity and have not necessarily been drawn to scale. For example, the dimension of some of the elements in the figures may have been exaggerated relative to other elements to help improve the understanding of the embodiments of the present invention. Identical numeral references in the figures refer to the same element.

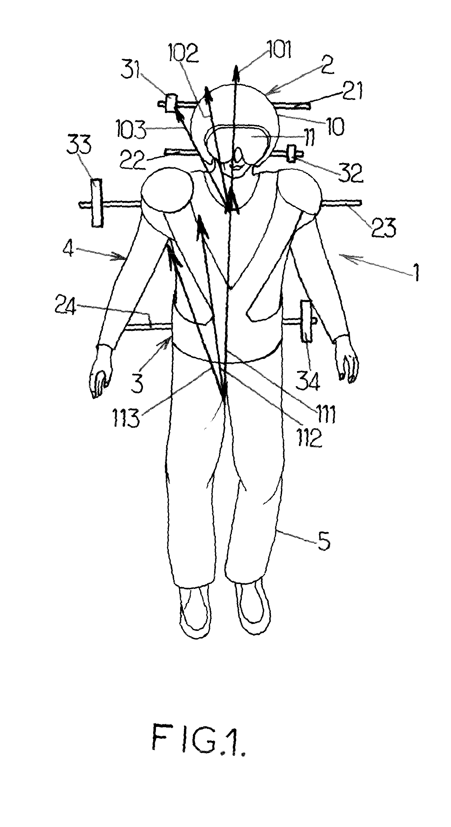

Figure 1 shows a standing person 1 equipped with a plurality of mass holding means 21, 22, 23, 24 attached to different parts of the body of the person. Mass holding means 21 and 22 are attached to the head 2 of the person thanks to a virtual reality helmet 10. Mass holding means 23 and 24 are attached to the upper part and the lower part of the trunk 3 of the person.

Each mass holding means 21, 22, 23, 24 comprises a bar which is suitable to receive weights, respectively weights 31, 32, 33, 34; said weights are movable along said bars and can be fixed in a desired position.

The virtual reality helmet 10 comprises a part surrounding the head 2 on which bars are fixed and a screen 11 on which virtual images can be projected.

According to an embodiment of the present invention where the non visual frame of reference to be tested is a proprioceptive frame of reference, the exocentered spatial mass repartition of the parts of the body of the person may be modified according to three types of axis :

- geometrical axis 101, 111, which relates to the distribution of the mass of the parts of the body, thus to the articular geometry of said body;

- mass center axis 102, 112, which relates to first moment linked to the rotation axis passing through the mass center of the distribution of mass of the body;

- inertia axis (called e3), 103, 113, which relates to second moment linked to inertial rotation axis of the distribution of the mass of the body and thus relates to dynamic components.

When only the head moves, axis 101, 102, 103 have to be considered.
When the whole body moves, axis 111, 112, 113 have to be considered and relates to the head 2, trunk 3 and legs 5 system.

In order to modify the proprioceptive frame of reference of the head and to let vary the spatial position of axis 101, 102, 103, the positions of weights 31 and 32 are modified.

In order to modify the proprioceptive frame of reference of the whole body and to let vary the spatial position of axis 111, 112, 113, the positions of weights 31, 32, 33 and 34 are modified.

Modification of the proprioceptive frame of reference can also results from moving a limb, such as an arm 4 or a leg 5.

Examples of modifications of a proprioceptive frame of reference and their relationship to the perception of a part of a body are disclosed in following publications:

- *"Perception of limb orientation in the vertical plane depends on center of mass rather than inertial eigenvectors"* - Rolf van de Langenberg ; Idsart Kingma ; Peter J. Beek - Exp. Brain Res (2007) 180:595-607*;*

- *"Differential exploitation of the inertia tensor in multi-joint arm reaching"* - Delphine Bernardin ; Brice Isableu ; Paul Fourcade ; Benoit G. Bardy - Exp Brain Res (2005) 167:487-495*)*.

Examples of methods for measuring the value of a parameter representative of the perception of spatial orientation are given in said publications, such as measuring the difference in limbs orientation or when pointing at a target according to different exocentered spatial mass repartitions of the parts of the body.

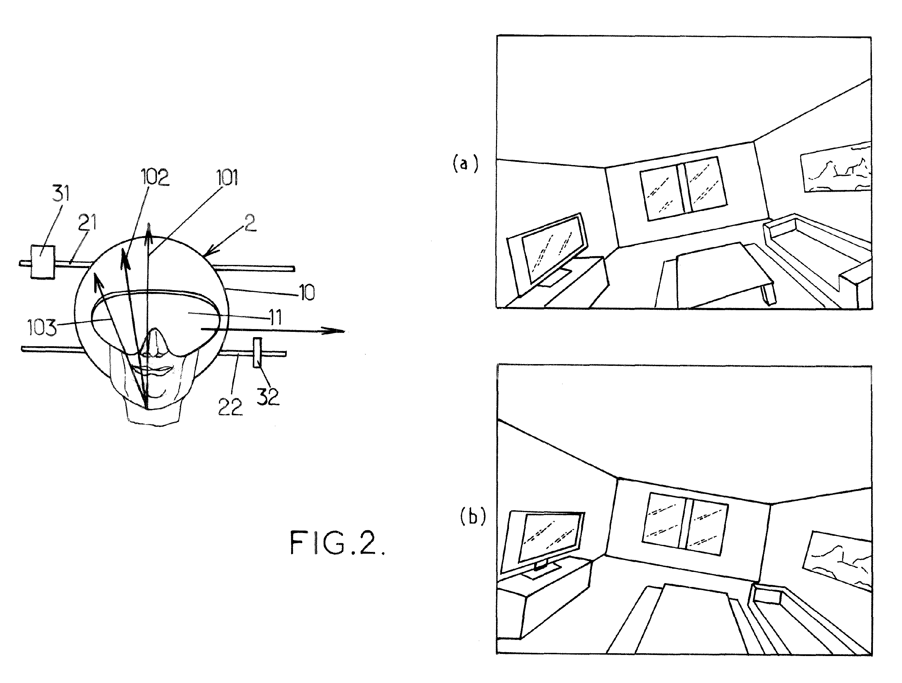

According to an embodiment of the present invention, and as illustrated in figure 2 a moving visual scene is provided to the person on the screen 11 of the virtual reality helmet 10. The horizontal arrow on figure 2 indicates the view on the helmet which is a continuously moving architectural scene from image (a) to image (b) and reverse. Such an architectural scene comprises lines that are recognizable by the person as horizontal and vertical lines.

The person is then asked to determine the perceived vertical or horizontal line.

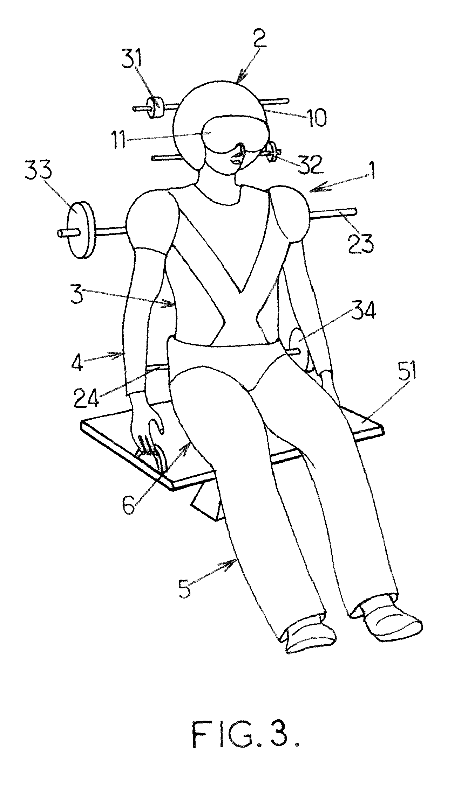

According to another embodiment illustrated on figure 3, the person is sitting and his buttock 6 is placed on a moving board 51 so as to modify the proprioceptive frame of reference corresponding to the gluteal support.

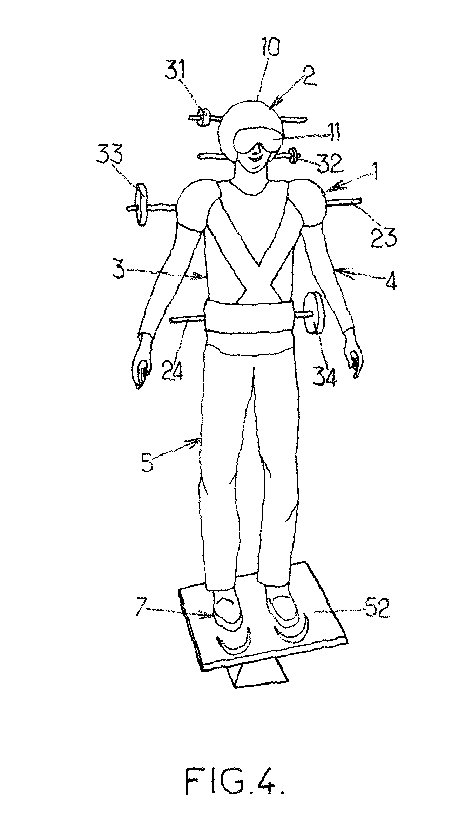

According to the embodiment illustrated on figure 4, the person is standing and his feet 7 are placed on a moving board 52 so as to modify the proprioceptive frame of reference corresponding to the feet support.

The moving board 51, 52 may be mechanized so as to precisely control its position variations.

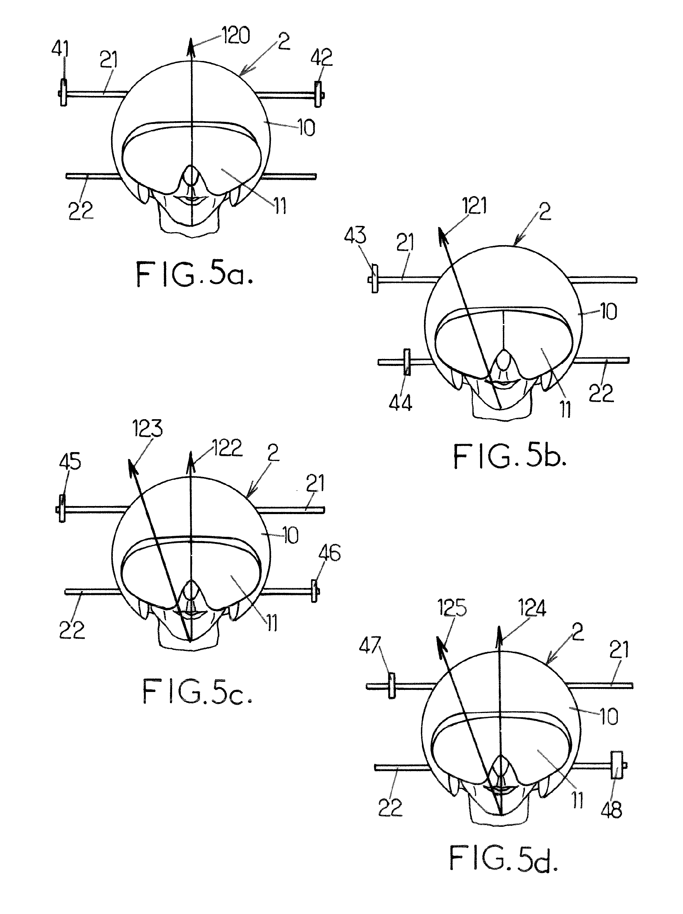

The figures 5a to d show different configurations of a virtual reality helmet 10 according to the present invention where the weights 41 to 48 are placed along the bars 21 and 22 in different positions in order to test the sensitivity of the person to different variations of the proprioceptive frame of reference, namely according to different rotation axes of the egocentric frame of reference..

In figure 5a, the weights 41 and 42 are equal and placed symmetrically to the geometrical axis on the same bar 21. The direction 120 thus corresponds to geometrical axis, to the mass center axis and to the inertia e3 axis. According to this embodiment, these three axes are merged together.

In figure 5b, both weights 43 and 44 are equal and placed at the right side of the head, respectively on the upper bar 21 and the lower bar 22. According to this embodiment, the direction 121 corresponds both to the mass center axis and to the inertia e3 axis. According to this embodiment, these two axes are merged together and the geometrical axis remains as represented in figure 5a.

In figure 5c, the weights 45 and 46 are equal and placed at the same distance of the geometrical axis, but weight 45 is placed on the upper bar 21 and weight 46 is placed on the lower bar 22. Direction 122 corresponds to the mass center axis and direction 123 corresponds to the inertia e3 axis. According to this embodiment, the geometrical axis and the mass center axis are merged together.

In figure 5d, the weight 48 is significantly heavier than weight 47 and the weight positions according to bars 21 and 22 are similar to the ones of figure 5c. Direction 124 corresponds to the inertia e3 axis and direction 125 corresponds to the mass center axis. According to this embodiment, the geometrical axis and the inertia e3 axis are merged together.

Each of the figures 1, 3, 4 and 5a to d embodiments corresponds to testing different main characteristics of the body and/or of the head proprioceptive frame of reference. Combining said variations of the proprioceptive frame of reference with variations of the visual frame of reference makes possible to determine the sensitivity of the person to combined visual and non visual frame of reference variations. According to the present invention, said sensitivity is referred to a scale of sensitivity to perception of spatial orientation. It is then possible to measure the perception of spatial orientation of a person in complex visual / non visual situations corresponding for example to virtual reality sickness, transport sickness, pilots' behaviour, sport training, and handicap rehabilitation.

The invention has been described above with the aid of embodiments without limitation of the general inventive concept. In particular the present invention provides a method for measuring the perception of spatial orientation in complex situations not limited to visual/proprioceptive frames of reference modifications, but also to numerous visual/non visual frames of reference modifications. Furthermore, a plurality of non visual frames of reference may be modified step by step or simultaneously.

**Claims**

1. A method for measuring the perception of spatial orientation of a person comprising the steps of:

    a1) measuring the value of a parameter representative of the perception of spatial orientation when the person's body is arranged according to a first non visual frame of reference;
    a2) modifying the first non visual frame of reference and thus providing a second non visual frame of reference and measuring the value of the parameter representative of the perception of spatial orientation;
    b1) measuring the value of the parameter representative of the perception of spatial orientation when the person's eyes are provided with a first visual frame of reference;
    b2) modifying the first visual frame of reference so as to provide a second visual frame of reference and measuring the value of the parameter representative of the perception of spatial orientation;
    c) combining the measured values of the parameters representative of the perception of spatial orientation of steps a1), a2), b1), b2) so as to obtain a value representing the sensitivity of the person referred to a scale of sensitivity to perception of spatial orientation.

2. The method of preceding claim wherein the non visual frame of reference is selected in the list consisting of a proprioceptive frame of reference, a vestibular frame of reference, a non visual prior knowledge frame of reference, an auditory frame of reference.

3. The method of preceding claim wherein the non visual frame of reference is a proprioceptive frame of reference and where modifying a proprioceptive frame of reference comprises modifying an exocentered spatial mass repartition of the parts of the body of the person.

4. The method of preceding claim wherein the variation of the exocentered spatial mass repartition of the parts of the body of the person is a variation between two static positions.

5. The method of claim 3 wherein the variation of the exocentered spatial mass repartition of the parts of the body of the person is a dynamic variation.

6. The method of any of preceding claims wherein the visual frame of reference is provided by projecting a three dimensional virtual visual scene on a screen.

7. The method of any of preceding claims wherein the three dimensional virtual visual scene comprises lines that are recognizable by the person as horizontal or vertical lines and wherein the parameter representative of the perception of spatial orientation is the difference between at least a perceived and an actual vertical line.

8. The method of any of preceding claims wherein the measuring steps of steps a1) and a2) are implemented without providing any visual scene to the person.

9. The method of any of preceding claims wherein the steps a1) and a2) are implemented with the same visual scene provided to the person, the steps b1) and b2) are implemented with the same non visual frame of reference provided to the person and the combination of measured values of step c) is implemented according to a multisensory integration model.

10. The method of any of claims 1 to 7 wherein the steps a2) and b2) are implemented simultaneously.

11. The method according to claim 8 combined with claim 9 and 10 wherein the result of the combination of the measured values of step c) according to claims 8 and 9 is further compared with the result of the combination of the measured values of step c) according to claim 10 so as to test, and possibly correct, the result of the multisensory integration model.

12. The method according to any of preceding claims wherein the scale of sensitivity to perception of spatial orientation is a numeral scale where the higher is the value representing the sensitivity of the person, the higher is the visual dependence compared to the non visual dependence according to the non visual frame of reference of steps a1), a2).

13. A virtual reality helmet comprising a projection device wherein bars are fixed on said helmet and are suitable to receive weights.

14. A computer program product comprising one or more stored sequences of instructions that are accessible to a processor and which, when executed by the processor, causes the processor to carry out the steps of any of claims 1 to 12.

15. A computer-readable medium carrying one or more sequences of instructions of the computer program product of preceding claim

FIG.1.

(a)

(b)

FIG.2.

FIG.3.

FIG.4.

FIG.5a.

FIG.5b.

FIG.5c.

FIG.5d.

Europäisches
Patentamt

European
Patent Office

Office européen
des brevets

## EUROPEAN SEARCH REPORT

Application Number

EP 08 30 5974

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 4 738 269 A (NASHNER LEWIS M [US]) 19 April 1988 (1988-04-19) | 1,8,10, 12,15 | INV. A61B3/032 A61B5/103 A61B5/11 |
| Y | * column 8, line 24 - column 9, line 34; claim 6; figures 1,2; table II * | 2,6,7,14 | |
| Y | US 5 303 715 A (NASHNER LEWIS M [US] ET AL) 19 April 1994 (1994-04-19) * column 10, line 64 - column 11, line 17; figure 1 * * column 12, line 33 - line 57 * | 2,14 | |
| Y | BAGUST ET AL: "Assessment of Verticality Perception by a Rod-and-Frame Test: Preliminary Observations on the Use of a Computer Monitor and Video Eye Glasses" ARCHIVES OF PHYSICAL MEDICINE AND REHABILITATION, W.B. SAUNDERS, vol. 86, no. 5, 1 May 2005 (2005-05-01), pages 1062-1064, XP005523861 ISSN: 0003-9993 * paragraph [METHODS]; figure 1 * | 7 | |
| Y | US 6 364 845 B1 (DUFFY CHARLES J [US] ET AL) 2 April 2002 (2002-04-02) * column 10, line 40 - line 55; figures 1A-D * | 6 | TECHNICAL FIELDS SEARCHED (IPC) A61B |
| X | DE 10 2006 056009 A1 (HEMAR EDUARD [DE]) 14 June 2007 (2007-06-14) * paragraph [0009] - paragraph [0015]; figures 1,5 * | 13 | |
| A | US 6 077 237 A (CAMPBELL CRAIG [US] ET AL) 20 June 2000 (2000-06-20) * column 3, line 23 - line 36; figure 3 * * column 4, line 27 - line 44 * | 1,13 | |

-/--

The present search report has been drawn up for all claims

| Place of search | Date of completion of the search | Examiner |
|---|---|---|
| Munich | 5 June 2009 | Schindler, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 08 30 5974

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 5 347 400 A (HUNTER KEN [US]) 13 September 1994 (1994-09-13) * column 5, line 18 - line 49; figures 1,2 * | 13 | |

-----

TECHNICAL FIELDS
SEARCHED      (IPC)

The present search report has been drawn up for all claims

| Place of search | Date of completion of the search | Examiner |
|---|---|---|
| Munich | 5 June 2009 | Schindler, Martin |

EPO FORM 1503 03.82 (P04C01)

Europäisches
Patentamt

European
Patent Office

Office européen
des brevets

Application Number

EP 08 30 5974

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

Application Number

EP 08 30 5974

The Search Division considers that the present European patent application does not comply with the
requirements of unity of invention and relates to several inventions or groups of inventions, namely:

```
1. claims: 1-12,14-15

        Method for measuring the perception of spatial orientation
                           ---

2. claim: 13

                    A virtual reality helmet
                           ---
```

**EP 2 198 770 A1**

**ANNEX TO THE EUROPEAN SEARCH REPORT
ON EUROPEAN PATENT APPLICATION NO.**

EP 08 30 5974

This annex lists the patent family members relating to the patent documents cited in the above-mentioned European search report.
The members are as contained in the European Patent Office EDP file on
The European Patent Office is in no way liable for these particulars which are merely given for the purpose of information.

05-06-2009

| Patent document cited in search report | | Publication date | Patent family member(s) | | Publication date |
|---|---|---|---|---|---|
| US 4738269 | A | 19-04-1988 | US | 5052406 A | 01-10-1991 |
| US 5303715 | A | 19-04-1994 | NONE | | |
| US 6364845 | B1 | 02-04-2002 | NONE | | |
| DE 102006056009 | A1 | 14-06-2007 | NONE | | |
| US 6077237 | A | 20-06-2000 | NONE | | |
| US 5347400 | A | 13-09-1994 | NONE | | |

EPO FORM P0459

For more details about this annex : see Official Journal of the European Patent Office, No. 12/82

19

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- Oltman PK. A portable rod-and-frame apparatus. *Percept. Mot. Skills,* 1968, vol. 26, 503-6
- Psychol. Lab., Dept. Psychiat. Downstate Medical Center
- **Witkin HA ; Goodenough DR ; Oltman P K.** Psychological differentiation: current status. *J. Personal. Soc. Psychol.,* 1979, vol. 37, 1127-45

- **Rolf van de Langenberg ; Idsart Kingma ; Peter J. Beek.** Perception of limb orientation in the vertical plane depends on center of mass rather than inertial eigenvectors. *Exp. Brain Res,* 2007, vol. 180, 595-607
- **Delphine Bernardin ; Brice Isableu ; Paul Fourcade ; Benoit G. Bardy.** Differential exploitation of the inertia tensor in multi-joint arm reaching. *Exp Brain Res,* 2005, vol. 167, 487-495